(19) **Europäisches Patentamt**

**European Patent Office**

**Office européen des brevets**

(11) Veröffentlichungsnummer: **0 306 709 B1**

(12) **EUROPÄISCHE PATENTSCHRIFT**

(45) Veröffentlichungstag der Patentschrift: **23.12.92**

(51) Int. Cl.⁵: **A61B 17/58**

(21) Anmeldenummer: **88112751.8**

(22) Anmeldetag: **05.08.88**

(54) **Markraum-Verriegelungsnagel.**

(30) Priorität: **05.09.87 DE 3729840**

(43) Veröffentlichungstag der Anmeldung:
**15.03.89 Patentblatt 89/11**

(45) Bekanntmachung des Hinweises auf die
Patenterteilung:
**23.12.92 Patentblatt 92/52**

(84) Benannte Vertragsstaaten:
**AT CH DE ES FR GB IT LI**

(56) Entgegenhaltungen:
**EP-A- 0 135 804**
**DE-A- 3 413 596**
**DE-U- 8 533 134**

(73) Patentinhaber: **Dauerer, Hermann Heinrich Michael**
**Brauhausstrasse 1**
**W-8531 Ipsheim(DE)**

(72) Erfinder: **Dauerer, Hermann Heinrich Michael**
**Brauhausstrasse 1**
**W-8531 Ipsheim(DE)**

(74) Vertreter: **Rau, Manfred, Dr. Dipl.-Ing. et al**
**Rau & Schneck, Patentanwälte Königstrasse 2**
**W-8500 Nürnberg 1(DE)**

## Beschreibung

Die Erfindung betrifft einen Markraum-Verriegelungsnagel nach dem Oberbegriff des Anspruches 1.

Bei einem derartigen durch die DE-PS 34 13 596 bekannten Markraum-Verriegelungsnagel ist der Innenkörper als sich annähernd über die gesamte Länge des Hohlkörpers erstreckendes Bauteil ausgebildet. Beim Einbringen des Hohlkörpers in den Markraum tritt in der Regel eine Verdrehung des Hohlkörpers um bis zu 90° ein. Eine solche Verdrehung führt zu einer Querschnittsverringerung des Hohlkörpers. Eine solche Querschnittsverringerung tritt auch noch durch die Verklemmung des Hohlkörpers im Knochen auf. Als Folge dieser Querschnittsverringerung ist es schwierig, den Innenkörper in den Hohlköper einzuführen. Es besteht die Gefahr, daß der Innenkörper im Bereich der Löcher beschädigt wird, da er in diesen Bereichen einen geschwächten Querschnitt aufweist.

Der Erfindung liegt die Aufgabe zugrunde, den Markraum-Verriegelungsnagel der gattungsgemäßen Art so auszugestalten, daß die beim Einbringen des Hohlkörpers in den Markraum auftretenden Querschnittsverringerungen das Einführen des Innenkörpers nicht behindern.

Diese Aufgabe wird bei einem Markraum-Verriegelungsnagel nach dem Oberbegriff des Anspruches 1 durch die Merkmale im Kennzeichnungsteil des Anspruches 1 gelöst. Das Einbringen der beiden Teilkörper ist beim erfindungsgemäßen Markraum-Verriegelungsnagel ohne Schwierigkeiten durchführbar, weil die relativ kleinen Teilkörper nur eine relativ geringe Reibung gegenüber der Innenwand des Hohlkörpers aufweisen und sehr viel leichter den anatomisch bedingten Biegungen der Verriegelungsnägel folgen können. Sie können einzeln mithilfe eines Einführinstrumentes eingebracht werden. Insbesondere im Bereich der Löcher tritt hierbei wegen des deutlich geringeren Bewegungswiderstandes ein geringerer Druck auf, wodurch eine Beschädigung vermieden wird.

Die Erfindung ermöglicht auch die Weiterbildung nach Anspruch 2. Sind an wenigstens einem Ende des Markraum-Verriegelungsnagels die Löcher als Langlöcher ausgebildet, so kann der entsprechende Teilkörper sich zusammen mit den Schrauben um eine geringe Strecke im Hohlkörper hin- und herbewegen. Diese Microbewegung, die in der Medizin überlicherweise als Microdynamic bezeichnet wird, findet in ähnlicher Weise an der Frakturstelle statt und verstärkt dort den Wachstumsreiz.

Durch die vorteilhafte Weiterbildung nach Anspruch 3 wird erreicht, daß der distal gelegene Teilkörper bei Einführen bzw. Eintreiben in den Hohlkörper exakt in eine Lage kommt, in der die Löcher im Hohlkörper und die Löcher im Teilkörper einander überdecken.

Die Erfindung wird nachfolgend an einem Ausführungsbeispiel näher erläutert. Es zeigt

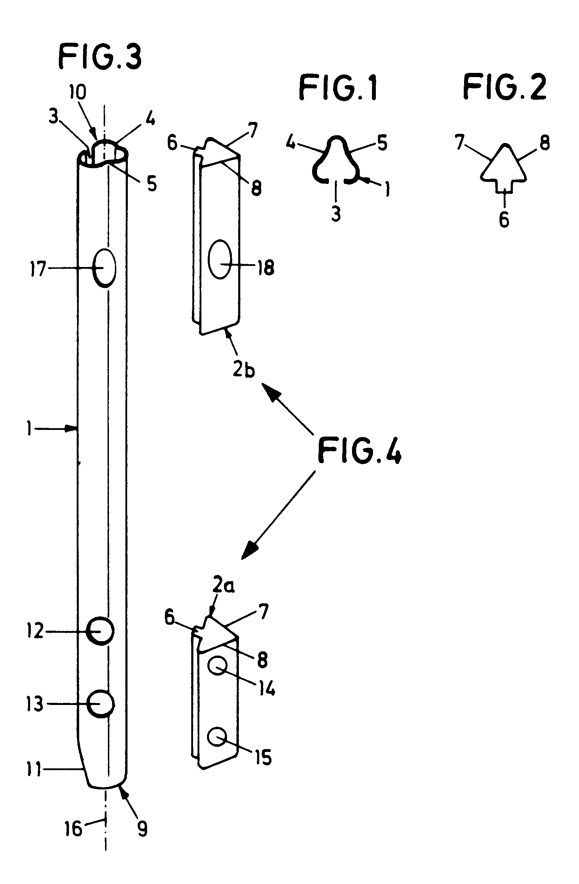

Fig. 1 eine Draufsicht auf den Hohlkörper,

Fig. 2 eine Draufsicht auf einen Teilkörper des Innenkörpers,

Fig. 3 eine perspektivische Seitenansicht des Hohlkörpers,

Fig. 4 eine perspektivische Seitenansicht der beiden Teilkörper des Innenkörpers in ihrer Längs-Zuordnung zum Hohlkörper nach Fig. 3. und

Fig. 5 eine Seitenansicht des Markraum-Verriegelungsnagels mit eingesetzten Schrauben.

Ein der in der Zeichnung dargestellter Markraum-Verriegelungsnagel weist einen Hohlkörper 1 und einen aus zwei Teilkörpern 2a, 2b bestehenden Innenkörper auf. Der Hohlkörper 1 weist einen - grob vereinfacht ausgedrückt - dreieckigen Querschnitt auf, wobei die Ekken abgerundet sind. In einer Längsseite weist der Hohlkörper 1 einen sich über seine volle Länge erstrekkenden Längsschlitz 3 auf. Die beiden anderen Längsseiten 4, 5 sind leicht nach innen gewölbt. Die Teilkörper 2a, 2b sind im Querschnitt identisch ausgebildet und in ihrer Grundform ebenfalls mit einem dreieckigen Querschnitt versehen, der dem Innenquerschnitt des Hohlkörpers 1 annähernd angepaßt ist. An einer Längsseite sind die Teilkörper 2a, 2b mit einem nach außen vorstehenden Längssteg 6 versehen, der in seiner Breite dem Längsschlitz 3 angepaßt ist, so daß die Teilkörper 2a, 2b im Hohlkörper 1 gegen Verdrehungen gesichert sind. Die beiden anderen Längsflächen 7, 8 sind den Längsseiten 4, 5 des Hohlkörpers 1 zugeordnet. Sie sind eben ausgebildet, so daß sie sich jeweils mit Sicherheit linienförmig aneinander anlegen. Durch die geschilderte Ausgestaltung ist sichergestellt, daß die Teilkörper 2a, 2b - jeweils bezogen auf den Querschnitt - mindestens eine Drei-Punkt-Anlage haben.

Im Bereich seiner beiden Enden 9, 10 ist der Hohlkörper 1 mit mehrerern Löchern versehen, die nachfolgend erläutert werden. Das in der Zeichnung unten dargestellte Ende des Hohlkörpers 1 ist sein distales Ende 9. Es handelt sich um das Ende, mit dem der Hohlkörper 1 zuerst in den Markraum des zu nagelnden Knochens eingeführt wird, daß also von der Einführstelle entfernt (distal) liegt. Bei dem in der Zeichnung oben dargestellten Ende handelt es sich um das proximale Ende, da es der Behandlungsstelle benachbart (proximal) liegt. Um das Einführen zu erleichtern, ist der Hohlkörper 1 am distalen Ende 9 mit einer Verjüngung 11 versehen.

Im Bereich des distalen Endes 9 wird der Teilkörper 2a angeordnet. Der Hohlkörper 1 und der Teilkörper 2a weisen hierzu in diesem Bereich sich durch die Längsseiten 4, 5 bzw. die Längsflächen 7, 8 erstreckende Löcher 12, 13 und 14, 15 auf, die senkrecht zur Längsachse 16 des Hohlkörpers 1 verlaufen. Die Löcher 12, 13 des Hohlkörpers 1 decken sich mit den Löchern 14, 15 im Teilkörper 2a, d.h. der Mittenabstand der Löcher ist identisch; beispielsweise beträgt er 30 mm. Der Durchmesser der Löcher 12, 13 im Hohlkörper 1 beträgt beispielsweise 6,1 mm. Hierzu zugeordnet beträgt dann der Durchmesser der Löcher 14, 15 im Teilkörper 2a etwa 5 mm. Der Mittenabstand des dem proximalen Ende 10 nähergelegenen Loches 12 des Hohlkörpers 1 vom proximalen Ende 10 beträgt beispielsweise 350 mm.

Dem proximalen Ende 10 des Hohlkörpers 1 benachbart ist ein Schrägloch 17 ausgebildet, das ebenfalls die Längsseiten 4, 5 des Hohlkörpers 1 durchsetzt. Die Neigung des Schrägloches 17 zur Längsachse 16 beträgt etwa 30°. Der mittlere Abstand des Schrägloches 17, also der Abstand der Mitte des Schrägloches 17 an der Schnittstelle mit der Längsachse 16 vom proximalen Ende 10 beträgt beispielsweise 40 mm. Auch hier ist der Bohrungsdurchmesser des Schrägloches 17 beispielsweise 6,1 mm. Diesem Schrägloch 17 ist ein mit der gleichen Neigung zur Längssachse 16 verlaufendes Schrägloch 18 im Teilkörper 2b zugeordnet, dessen Bohrungsdurchmesser beispielsweise ebenfalls 5,0 mm beträgt.

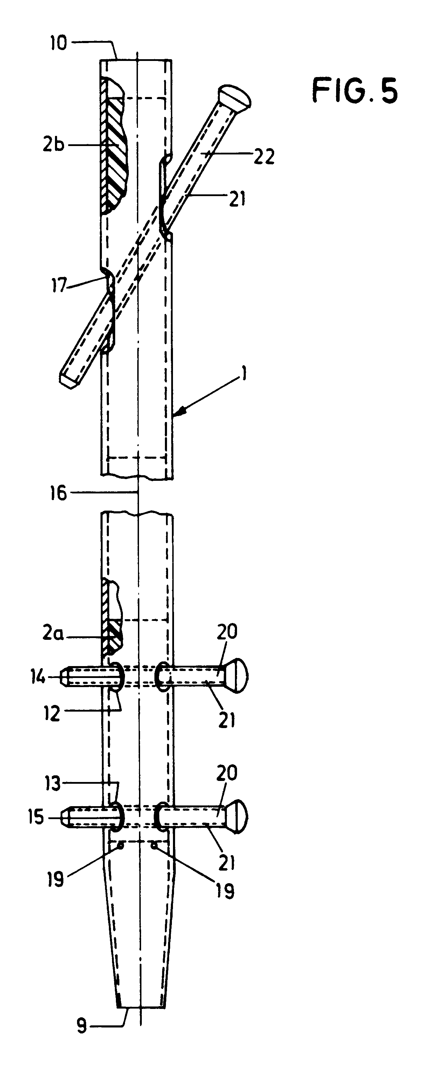

Aus Fig. 5 ist erkennbar, daß dem distalen Ende 9 des Hohlkörpers 1 benachbart in letzterem ein oder mehrere Anschläge 19 für den Teilkörper 2a vorgesehen sind. Wenn letzterer vom proximalen Ende 10 her in den Hohlkörper 1 eingetrieben wird, dann wird durch diesen Anschlag bzw. diese Anschläge 19 selbsttätig die Lage des Teilkörpers 2a so festgelegt, daß seine Löcher 14, 15 sich exakt mit den Löchern 12, 13 des Hohlkörpers 1 decken.

Wie ebenfalls Fig. 5 entnehmbar ist, wird durch die Loch-Paare 12, 14 und 13, 15 jeweils eine Schraube 20 hindurchgeschraubt, die über ihre volle Länge mit einem Außengewinde 21 versehen ist. Der Kerndurchmesser dieses Gewindes 21 entpricht etwa dem Bohrungsdurchmesser des jeweiligen Loches 14 bzw 15 im Teilkörper 2a, so daß sich das Gewinde 21 in diesen Teilkörper 2a einschneidet. Damit dies möglich ist, besteht der Teilkörper 2a gleichermaßen wie der Teilkörper 2b aus einem gewebeverträglichen Kunststoff, wie er beispielsweise von der Firma Ruhr Chemie unter dem geschützten Warenzeichen "CHIROLEN" vertrieben wird. Die Verformfestigkeit dieses Kunststoffes ist geringer als die des Metalls, aus dem der Hohlkörper 1 besteht. In dem gewählten Beispiel ist der Außendurchmesser des Gewindes 21 6 mm, so daß ein geringfügiges Spiel zwischen dem Gewinde 21 in den Löchern 12, 13 besteht, so daß also keine Späne von den aus gewebeverträglichem Metall bestehenden Hohlkörper 1 beim Einschrauben der Schrauben 20 abgeschnitten werden können. Andererseits ist beim Verschrauben eine völlig feste Verbindung zwischen dem Knochen und dem Hohlkörper 1 mit Teilkörper 2a gegeben. Für eine durch den Teilkörper 2b zu schraubende Schraube 22 gelten die gleichen Verhältnisse.

Wie ebenfalls Fig. 5 anschaulich zu entnehmen ist, ist das Schrägloch 17 im Hohlkörper 1 in geringem Umfang als Langloch ausgebildet, so daß der Teilkörper 2b nach dem Einschrauben einer Schraube 22 sich zusammen mit der Schraube 22 relativ zum Hohlkörper 1 in geringem Umfang in Richtung der Längsachse, beispielsweise um 0,5 bis 2 mm, bewegen kann. Eine solche geringe Längsbeweglichkeit kann auch in gleicher Weise für einen Teilkörper 2a mit zwei beispielsweise quer zur Längsachse 16 verlaufenden Löchern vorgesehen sein.

## Patentansprüche

1.  Markraum-Verriegelungsnagel zur Frakturbehandlung in langen Röhrenknochen, bestehend aus einem Hohlkörper (1) aus gebeverträglichem Metall, der mit Löchern (12, 13, 17) zur Aufnahme von Schrauben (20, 22), sowie mit einem Längsschlitz (3) versehen ist, und aus einem in dem Hohlkörper (1) befindlichen, ebenfalls gewebeverträglichen, aber nicht metallischen Innenkörper (2a, 2b), dessen Material eine niedrigere Verformfestigkeit aufweist als das des Hohlkörpers (1) und dessen Profil so gestaltet ist, daß der Innenkörper (2a, 2b) den Längsschlitz (3) des Hohlkörpers (1) ausfüllt und der Innenkörper (2a, 2b) den Hohlkörper (1) im Querschnitt an wenigstens 3 Punkten berührt, wobei der Innenkörper (2a, 2b) ebenfalls mit Löchern (14, 15, 18) versehen ist, die in Lage und Anordnung mit denen des Hohlkörpers (1) übereinstimmen und deren Durchmesser kleiner ist als der Durchmesser der Löcher (12, 13, 17) im Hohlkörper (1), dadurch gekennzeichnet, daß der Innenkörper aus zwei Teilkörpern (2a, 2b) besteht, die in ihrer Längsausdehnung so groß sind, wie es für das Halten von in ihre Löcher (14, 15, 18) eingebrachten Schrauben (20, 22) erforderlich ist.

2.  Verriegelungsnagel nach Anspruch 1, dadurch gekennzeichnet, daß das wenigstens eine dem wenigstens einen Teilkörper (2b) zugeordnete Loch (17) im Hohlkörper (1) als Langloch ausgebildet ist.

3. Verriegelungsnagel nach Anspruch 1, dadurch gekennzeichnet, daß der Hohlkörper (1) im Bereich seines dem Einführende (proximales Ende 10) für die Teilkörper (2a, 2b) entfernten Endes (distales Ende 9) mindestens einen Anschlag (19) für den zugeordneten Teilkörper (2a) aufweist.

## Claims

1. An intramedullary anchorage nail for the fracture treatment in long tubular bones, consisting of a hollow body made of a tissue tolerated metall, which is provided with holes (12, 13, 17) for locating screws (20, 22) as well as with a longitudinal slot (3), and of an equally tissue tolerated, but non-metallic inner body (2a, 2b), which is located in the hollow body (1), the material of which inner body (2a, 2b) has a lower deformation stability than that of the hollow body (1), and the profile of which is formed in such a manner that the inner body (2a, 2b) fills the longitudinal slot (3) of the hollow body (1) and that the inner body (2a, 2b) contacts the hollow body (1) in its cross-section at at least 3 points, the inner body (2a, 2b) being also provided with holes (14, 15, 18), which coincide in their position and arrangement with those of the hollow body (1) and the diameter of which holes (14, 15, 18) is smaller than the diameter of the holes (12, 13, 17) in the hollow body (1), characterized in that the inner body consists of two partial bodies (2a, 2b), which in their longitudinal extension are as large as is required for the retaining of screws (20, 22) placed in their holes (14, 15, 18).

2. An anchorage nail according to claim 1, characterized in that the at least one hole (17) associated to the at least one partial body (2b) is formed in the hollow body (1) as a longitudinal hole.

3. An anchorage nail according to claim 1, characterized in that in the area of its end (distal end 9), which is remote from the insertion end (proximal end 10) for the partial body (2a, 2b), the hollow body (1) comprises at least one stop (19) for the associated partial body (2a).

## Revendications

1. Clou de verrouillage intramédullaire pour le traitement de fractures dans les os longs tubulaires, constitué par un corps creux (1) qui est réalisé en un métal présentant une bonne compatibilité avec les tissus, et qui est pourvu d'une fente longitudinale (3) ainsi que de trous (12, 13, 17) destinés à recevoir des vis (20, 22), et par un corps intérieur (2a, 2b) se trouvant dans le corps creux (1) et présentant également une bonne compatibilité avec les tissus, mais non métallique, dont le matériau présente une résistance à la déformation moindre que celui du corps creux (1), et dont le profil est d'une configuration telle que le corps intérieur (2a, 2b) comble la fente longitudinale (3) du corps creux (1) et que le corps intérieur (2a, 2b) touche le corps creux (1), en section transversale, en au moins trois points, le corps intérieur (2a, 2b) étant également pourvu de trous (14, 15, 18) qui correspondent en position et en disposition à ceux du corps creux (1), et dont le diamètre est inférieur au diamètre des trous (12, 13, 17) dans le corps creux (1), caractérisé en ce que le corps intérieur est composé de deux corps partiels (2a, 2b) dont l'étendue longitudinale est d'une grandeur égale à celle qui est nécessitée pour assurer le maintien des vis (20, 22) introduites dans leurs trous (14, 15, 18).

2. Clou de verrouillage selon la revendication 1, caractérisé en ce que ledit au moins un trou (17) dans le corps creux (1), associé au dit au moins un corps intérieur (2b), est réalisé sous la forme d'un trou oblong.

3. Clou de verrouillage selon la revendication 1, caractérisé en ce que le corps creux (1), dans la zone de son extrémité (extrémité distale 9) éloignée de l'extrémité d'introduction (extrémité proximale 10) pour les corps partiels (2a, 2b), présente au moins une butée (19) pour le corps partiel (2a) associé.

FIG.3

FIG.1

FIG.2

FIG.4

FIG.5